(19) Europäisches Patentamt
European Patent Office
Office européen des brevets

(11) Veröffentlichungsnummer: **0 336 216 B1**

(12) # EUROPÄISCHE PATENTSCHRIFT

(45) Veröffentlichungstag der Patentschrift:
**27.12.91 Patentblatt 91/52**

(51) Int. Cl.[5]: **C07C 53/12**, C07C 51/54

(21) Anmeldenummer: **89105112.0**

(22) Anmeldetag: **22.03.89**

(54) **Verfahren zur Herstellung von Monocarbonsäureanhydriden.**

(30) Priorität: **02.04.88 DE 3811343**

(43) Veröffentlichungstag der Anmeldung:
**11.10.89 Patentblatt 89/41**

(45) Bekanntmachung des Hinweises auf die Patenterteilung:
**27.12.91 Patentblatt 91/52**

(84) Benannte Vertragsstaaten:
**AT BE CH DE FR GB IT LI NL**

(56) Entgegenhaltungen:
**EP-A- 0 203 286**

(73) Patentinhaber: **HOECHST AKTIENGESELLSCHAFT**
**Postfach 80 03 20**
**W-6230 Frankfurt am Main 80 (DE)**

(72) Erfinder: **Luft, Gerhard, Prof. Dr.**
**Ludwigstrasse 141a**
**W-6109 Mühltal (DE)**
Erfinder: **Trabold, Peter, Dr.**
**Ahornweg 19a**
**W-6110 Dieburg (DE)**

EP 0 336 216 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400°C und Drücken von 1-150 bar, wobei im Trägerkatalysator das Trägermaterial eine Edelmetall-Chelatverbindung trägt, die aus einer Edelmetallverbindung aus der Gruppe VIII des Periodensystems und einem Chelator mit Organostickstoff-, Organophosphor-, Organoarsen-, Organoschwefel- oder Mercaptogruppen gebildet ist.

Ein derartiges, in der Gasphase mit einem Trägerkatalysator arbeitendes Verfahren ist bereits aus der DE-OS 3511050 bekannt, welche die bei den Flüssigphaseverfahren auftretenden Nachteile, z.B. die schwierige Abtrennung und Rückführung von suspendiertem und teilweise gelöstem Katalysator und ggf. Promotor, vermeidet.

Es ist Aufgabe der vorliegenden Erfindung, den Chelator so zu modifizieren, daß sich Standzeit (Aktivitätsdauer) und Selektivität des Trägerkatalysators bei gleichem Trägermaterial deutlich verbessern.

Im einzelnen ist das Verfahren der Erfindung dadurch gekennzeichnet, daß

1. der Chelator in seinem Grundkörper mit Alkyl-, Aryl- oder Aralkylgruppen substituiert ist und eine der folgenden Strukturformeln aufweist:

a) 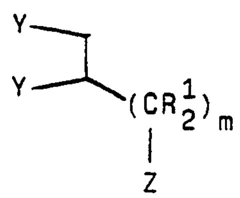

b) 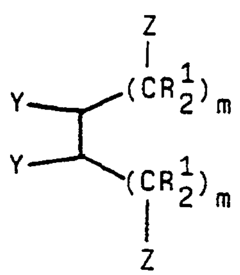

wobei

Y = $-NR^2_2$, ein stickstoffhaltiger Arylrest, $-PR^2_2$, $-AsR^2_2$, $-SR^2$ oder $-SH$ ;
Z = $-H$, Aryl, Phenyl (ggf. ortho-, meta- oder parasubstituiert)
$R^1$ = $-H$, $C_1$ bis $C_3$-Alkyl ;
$R^2$ = $C_1$ bis $C_6$-Alkyl, $C_5$ oder $C_6$-Cycloalkyl, $-C_6H_5$ oder $C_6H_5CH_2-$, die ggf. mit Halogen-, Methoxy-, Ethoxy- oder $C_1$ bis $C_3$-Alkylgruppen substituiert sind ;
m = 2-6, vorzugsweise 2-4.

Darüberhinaus kann das Verfahren der Erfindung wahlweise und bevorzugt dadurch gekennzeichnet sein, daß

2. im Trägerkatalysator das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator nach Punkt 1 gebildet ist.

3. der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält.

4. der Trägerkatalysator ein anorganisches oxidisches Trägermaterial oder einen Aktivkohleträger enthält.

5. der Trägerkatalysator zusammen 0.01 bis 50 Gew%, vorzugsweise 0.1 bis 20 Gew%, Chelatverbindungen und ggf. Nichtedelmetallverbindungen enthält.

6. der Trägerkatalysator in einer Korngröße von 1 bis 20 mm eingesetzt wird.

Als Katalysatorträger kommen bevorzugt anorganische Oxide, wie z.B. $SiO_2$, $Al_2O_3$, MgO, $TiO_2$, $La_2O_3$,

$ZrO_2$, Zeolith, Ton, NiO, $Cr_2O_3$, $WO_3$ oder entsprechende Mischoxide, aber auch Aktivkohle in Frage, welche BET-Oberflächen von 1-1000 $m^2/g$, vorzugsweise 30-400 $m^2/g$, haben.

Ebenso wie gemäß DE-Offenlegungsschrift 3511050 sind die Promotoren der 5. oder 6. Hauptgruppe in den erfindungsgemäß eingesetzten Chelatoren bzw. Chelatliganden chemisch gebunden. Sie bilden selbst eine funktionelle Gruppe, die die Edelmetallverbindungen aus der Gruppe VIII und ggf. Nichtedelmetallverbindungen aus der 6. oder 8. Nebengruppe chelatisieren. Es ist ein Vorteil, daß die zur Steigerung der Aktivität und der Selektivität der Trägerkatalysatoren notwendigen Promotoren aus der 5. oder 6. Hauptgruppe des Periodensystems der Elemente eine funktionelle Gruppe Y in den Chelatoren bilden und somit fixiert sind, weil dadurch eine Abtrennung und Rückführung dieser z.B. Organostickstoff- oder Organophosphorpromotoren entfällt.

Das Verfahren der Erfindung zur Herstellung von Monocarbonsäureanhydriden weist gegenüber dem Verfahren der DE-OS 3511050 höhere Selektivitäten und besonders bei Langzeitbelastung höhere Standzeiten des Trägerkatalysators auf.

Ein weiterer Vorteil des Verfahrens der Erfindung ist darin zu sehen, daß die auf dem Trägermaterial aufgetragenen modifizierten Edelmetall-Chelatverbindungen und ggf. Nichtedelmetall-Chelatverbindungen noch höhere Schmelzpunkte (240-270°C) als die in der DE-OS 3511050 beschriebenen Komplexe besitzen, was zu einer höheren thermischen Stabilität der Katalysatoren bzw. zu einer Erhöhung des Anwendungsbereiches von 20 bis 50°C führt.

Das Verfahren der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1-4 C-Atomen darstellt.

Die Trägermaterialien wurden bereits genannt; als Mischoxide kommen zB. $Cr_2O_3$—$Al_2O_3$, $WO_3$—$Al_2O_3$, MgO—$Al_2O_3$, $SiO_2$—$Al_2O_3$ oder $ZrO_2$—$Al_2O_3$ in Frage. Der Trägerkatalysator enthält bevorzugt 0.05 bis 5 Gew% Edelmetall.

Als Edelmetallverbindungen können bei der Herstellung des Trägerkatalysators z.B. folgende Verbindungen eingesetzt werden :

Rhodium :
$RhCl_3$, $RhCl_3 \cdot 3\ H_2O$, $RhBr_3$, $RhI_3$, $Rh(NO_3)_3$, $Rh_2(CO)_4Cl_2$, $Rh_2(CO)_4Br_2$, $Rh(CO)_4I_2$, $[P(C_6H_5)_3]_3RhCl$, $[P(C_6H_5)_3]_2Rh(CO)Cl$, $Rh_6(CO)_{16}$, $Rh_4(CO)_{12}$, $Rh_2(O_2CCH_3)_4$, $[RhCl(C_8H_{12})]_2$ ;

Iridium :
$IrCl_3$, $[Ir(CO)_3Cl]_2$, $Ir[P(C_6H_5)_3]_2(CO)Cl$, $Ir_4(CO)_{12}$, $[IrCl(C_8H_{12})]_2$, $Cl(CO)_2Irpyr$ (pyr = $C_6H_5N$) ;

Palladium :
$PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)_2PdCl_2$ ;

Ruthenium :
$RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, CO, die ebenfalls mit den Chelatoren reagieren, kommen z.B. ferner in Frage :

Chrom :
$Cr(CO)_6$, $CrCl_3$, $C_7H_8Cr(CO)_3$.

Nickel :
$Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Periodensystems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle können zusätzlich, z.B. als Lösung durch Imprägnierung, auf den Katalysatorträger aufgebracht werden.

Zur Herstellung des erfindungsgemäß eingesetzten Trägerkatalysators muß zuerst der Chelator mit den funktionellen Gruppen Y bereitgestellt werden. Dieser kann nach oder in Analogie zu Literaturangaben dargestellt werden. Im allgemeinen wird dann eine der genannten Edelmetallverbindungen aus der Gruppe VIII und ggf. eine der genannten Nichtedelmetallverbindungen aus der 6. oder 8. Nebengruppe in Lösung mit dem Chelator in Verbindung gebracht, wobei Chelatverbindungen entstehen, deren Schmelzpunkte über der Temperatur der Carbonylierungsreaktion zur Herstellung von Monocarbonsäureanhydriden liegen.

Anschließend erfolgt die Imprägnierung des Trägermaterials mit den gelösten Chelatverbindungen zum fertigen Trägerkatalysator. Die Lösemittel für die Chelatverbindungen können polar oder unpolar sein, z.B. Dichlormethan, Chloroform, Methanol, Benzol, Toluol oder Xylol, worin das Trägermaterial suspendiert wird.

Alle weiteren Einzelheiten zu Synthesen ergeben sich aus der Beschreibung der Katalysatorherstellung.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 mol, vorzugsweise 1 bis 100 mol, je 1 mol Jod(verbindung) oder Brom(verbindung). Die Temperatur der Reaktionszone wird so gewählt, daß das Reaktionsgemisch bei jedem beliebigen Umsatz gasförmig vorliegt. Bevorzugt wird die Temperatur zwischen 150 und 250°C gewählt. Der bevorzugte Druck liegt zwischen 5 und 30 bar.

Die Verweilzeit des Reaktionsgemisches am festen Trägerkatalysator beträgt 1 bis 1000 s, bevorzugt 1 bis 180 s. Die Umsetzung kann in einem senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter wasserfreien Bedingungen durch, doch sind geringe Wassermengen, wie sie in den handelsüblichen Ausgangsstoffen vorkommen, zulässig, sollten aber 1 mol%, berechnet auf die Ausgangsstoffe, nicht übersteigen. Auch wird die Carbonylierung durch geringe Mengen Methanol in den Ausgangsstoffen nicht beeinträchtigt. Ebensowenig stört Wasserstoff, der in kleinen Mengen im handelsüblichen Kohlenmonoxid vorhanden sein kann.

Das aus der Carbonylierungszone abströmende Reaktionsgemisch ist gasförmig und enthält Kohlenmonoxid, Methyljodid, Essigsäureanhydrid, nichtumgesetztes Methylacetat oder Dimethylether und ggf. sehr geringe Mengen Essigsäure.

Das gasförmige Reaktionsgemisch wird abgekühlt, Essigsäureanhydrid und ggf. Essigsäure auskondensiert und die nicht kondensierten Stoffe wie CO, Methyljodid, Methylacetat oder Dimethylether in die Reaktionszone zurückgeführt. Die umgesetzten Anteile von Ester oder Ether sowie CO werden fortlaufend ersetzt.

Die einfache Abtrennung der Anhydride durch Kühlung des abströmenden Reaktionsgemisches und Rückführung der nicht kondensierbaren Gase stellt, wie beim Verfahren der DE-OS 3511050, einen wesentlichen Vorteil dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

## Beispiele

### Rührautoklavversuche

Man verwendet einen 0.25 Liter fassenden Rührautoklaven aus korrosionsfreiem Edelstahl (Hastelloy C) mit den erforderlichen Zu- und Ableitungen, der einen drehbaren Katalysatorkorb enthält.

Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt.

Der Autoklav wird mit 2.5 ml eines flüssigen Gemisches aus 20 Volumenteilen Methyljodid und 80 Volumenteilen Ester oder Ether beschickt und auf Reaktionstemperatur aufgeheizt. Die Carbonylierung wird durch Aufpressen von Kohlenmonoxid eingeleitet. Der CO-Druck wird durch regelmäßiges Nachdrücken konstant gehalten.

Die Einzelheiten der Versuchsdurchführungen ergeben sich aus den Beispielen.

### Beispiel 1

2 ml (1.86 g) Essigsäuremethylester, 0.5 ml (1.14 g) Methyljodid und 6.46 g Katalysator Nr. 1 werden im Autoklaven mit Kohlenmonoxid bei 15 bar CO-Druck und 180°C umgesetzt. Nach 1 h Reaktionszeit ergibt sich eine Katalysatorleistung von 23 g $Ac_2O/g_{Rh} \cdot h$, bei einer Selektivität von 98%.

### Beispiel 2

2 ml (1.86 g) Essigsäuremethylester, 0.5 ml (1.14 g) Methyljodid und 6.77 g Katalysator Nr. 2 werden im Autoklaven mit Kohlenmonoxid bei 15 bar CO-Druck und 180°C umgesetzt. Nach 1 h Reaktionszeit ergibt sich

eine Katalysatorleistung von 22 g $Ac_2O/g_{Rh} \cdot h$, bei einer Selektivität von 98%.

Beispiel 3

2 ml (1.86 g) Essigsäuremethylester, 0.5 ml (1.14 g) Methyljodid und 7.98 g Katalysator Nr. 3 werden im Autoklaven mit Kohlenmonoxid bei 15 bar CO-Druck und 180°C umgesetzt. Nach 1 h Reaktionszeit ergibt sich eine Katalysatorleistung von 28 g $Ac_2O/g_{Rh} \cdot h$, bei einer Selektivität von 96%.

Beispiel 4

2 ml (1.86 g) Essigsäuremethylester, 0.5 ml (1.14 g) Methyljodid und 5.04 g Katalysator Nr. 4 werden im Autoklaven mit Kohlenmonoxid bei 15 bar CO-Druck und 180°C umgesetzt. Nach 1 h Reaktionszeit ergibt sich eine Katalysatorleistung von 44 g $Ac_2O/g_{Rh} \cdot h$, bei einer Selektivität von 97%.

Beispiel 5

Ein Stahlrohr von 20 mm Durchmesser und 400 mm Länge wird als Strömungsrohr senkrecht angeordnet und mit 52.8 g Katalysator Nr. 1 gefüllt. Bei einem Druck von 12 bar und einer Temperatur von 180°C werden stündlich 8 Nl CO(Nl = Liter, gemessen bei 1.013 bar und 0°C), sowie ein verdampftes Gemisch (12.9 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 15.4 g $Ac_2O/g_{Rh} \cdot h$, bei einer Selektivität von 99%.

Unter diesen Reaktionsbedingungen wurde die Carbonylierung 280 Stunden durchgeführt, wobei der eingesetzte Trägerkatalysator keinen Aktivitätsverlust zeigte.

Beispiel 6

Ein Stahlrohr von 20 mm Durchmesser und 400 mm Länge wird als Strömungsrohr senkrecht angeordnet und mit 51.0 g Katalysator Nr. 2 gefüllt. Bei einem Druck von 12 bar und einer Temperatur von 180°C werden stündlich 8 Nl CO (Nl = Liter, gemessen bei 1.013 bar und 0°C), sowie ein verdampftes Gemisch (13.5 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 14.3 g $Ac_2O/g_{Rh} \cdot h$, bei einer Selektivität von 99%.

Unter diesen Reaktionsbedingungen wurde die Carbonylierung 280 Stunden durchgeführt, wobei der eingesetzte Trägerkatalysator keinen Aktivitätsverlust zeigte.

Beschreibung der Katalysatorherstellung

In allen Fällen wurden die Katalysatorträger zur Aktivierung zuvor bei 200°C und 0.1 mbar 10 h getrocknet. Nach Aufbringen der Metallkomponente erhitzte man die Katalysatoren 8 h mit Chlortrimethylsilan zum Sieden und trocknete anschließend bei 0.1 mbar und 100°C. Sämtliche Synthesen wurden in Argonatmosphäre unter Ausschluß von Luftsauerstoff und Wasser ausgeführt. Alle verwendeten Lösemittel wurden zuvor über Molekularsieb 4 A oder wenn möglich mit Benzophenonnatrium getrocknet.

Das in den nachfolgenden Formeln gebrauchte Symbol "Ø" steht für den Phenylrest ($C_6H_5$).

Katalysator Nr. 1

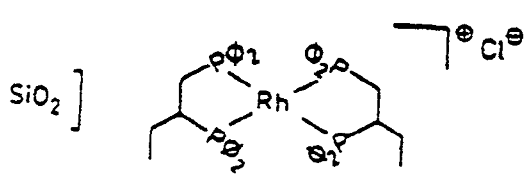

65.4 g aktivierte Siliciumdioxidpellets 1/8" × 1/8" (95% $SiO_2$) mit einer inneren Oberfläche nach BET von 68 $m^2/g$ und einem Porenvolumen von 0.43 ml/g wurden mit 150 ml einer Lösung aus 629 mg des Komplexes

4 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Pellets |
| Rh-Gehalt : | 0.1 Gew% |

Syntheseweg des Rhodiumkomplexes 4

1,2-Dichlorbutan (2) :

2 kann durch Umsetzung von 1-Buten (1) mit Chlor bei 0°C in Dichlormethan in fast quantitativer Ausbeute erhalten werden.

1,2-Bis-(diphenylphosphino)butan (3) :

3 wird durch Umsetzung doppeltmolarer Menge Natriumdiphenylphosphid in Dioxan mit 2, gelöst in Tetrahydrofuran, bei Raumtemperatur synthetisiert [analog 1,2-Bis(diphenylphosphino)ethan ; siehe K. Issleib und D.-W. Müller, Chem. Ber. 92, 3175 (1959)]. Ausbeute 78%.

[1,2-Bis(diphenylphosphino)butan]rhodium(I)-chlorid (4) :

Eine Lösung von 4 mmol 3 in Benzol wird unter Rühren zu einer Lösung von 1 mmol Dichlortetracarbonyldirhodium in Benzol getropft, wobei der Komplex 4 analysenrein ausfällt. Ausbeute 94%. Vgl. Synthese von [1,2-Bis(diphenylphosphino)ethan]rhodium(I)-chlorid : A. Sacco et al., J. Chem. Soc. (London), 3274 (1964).

Katalysator Nr. 2

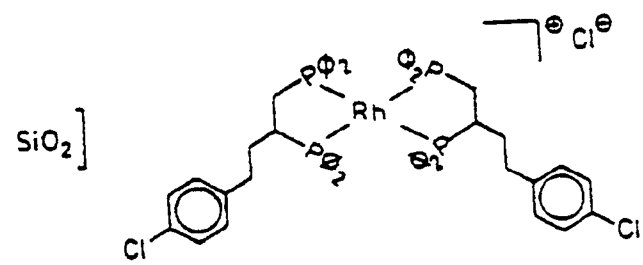

68.3 g aktivierte Siliciumdioxidpellets 1/8" × 1/8" (95% $SiO_2$) mit einer inneren Oberfläche nach BET von 68 $m^2/g$ und einem Porenvolumen von 0.43 ml/g wurden mit 150 ml einer Lösung aus 723 mg des Komplexes 8 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Pellets |
| Rh-Gehalt : | 0.09 Gew% |

Syntheseweg des Rhodiumkomplexes 8

1,2-Dichlor-4-(4-chlorphenyl)butan (6) :

6 kann durch Umsetzung von 4-(4-Chlorphenyl)buten (5) mit Chlor bei 0°C in Dichlormethan synthetisiert werden. Ausbeute 93%.

1,2-Bis(diphenylphosphino)-4-(4-chlorphenyl)-butan (7) :

7 wird durch Umsetzung doppeltmolarer Menge Natriumdiphenylphosphid in Dioxan mit 6, gelöst in Tetrahydrofuran, bei Raumtemperatur mit 82% Ausbeute synthetisiert [analog 1,2-Bis(diphenylphosphino)ethan ; siehe K. Issleib und D.-W. Müller, Chem. Ber. 92, 3175 (1959)].

[1,2-Bis(diphenylphosphino)-4-(4-chlorphenyl)butan]-rhodium(I)-chlorid (8) :

Eine Lösung von 4 mmol 7 in Benzol wird unter Rühren zu einer Lösung von 1mmol Dichlortetracarbonyldirhodium in Benzol getropft, wobei der Komplex 8 analysenrein ausfällt. Ausbeute 96%. Vgl. Synthese von [1,2-Bis(diphenylphosphino)ethan] rhodium(I)-chlorid ; A. Sacco et al., J. Chem. Soc. (London), 3274 (1964).

Katalysator Nr. 3

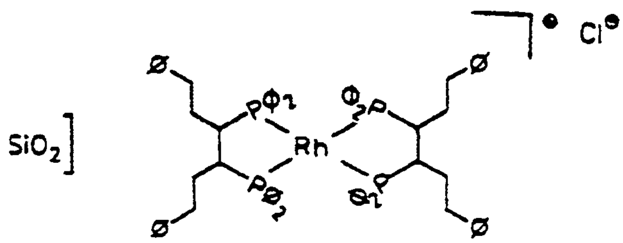

12.8 g aktivierte Siliciumdioxidpellets 1/8" × 1/8" (95% $SiO_2$) mit einer inneren Oberfläche nach BET von 68 $m^2/g$ und einem Porenvolumen von 0.43 ml/g wurden mit 50 ml einer Lösung aus 134.6 mg des Komplexes 12 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Pellets |
| Rh-Gehalt : | 0.08 Gew% |

Syntheseweg des Rhodiumkomplexes 12

1,6-Diphenylhex-3-en (9) :

Das Alken 9 wurde durch Umsetzung von Dihydrozimtaldehyd mit 3-Phenylpropyltriphenylphosphoniumbromid in 64%iger Ausbeute synthetisiert [Wittigreaktion ; siehe Organikum, 15. Aufl., S. 494, VEB Deutscher Verlag der Wissenschaften, Berlin 1977].

3,4-Dichlor-1,6-diphenylhexan (10) :

10 kann durch Umsetzung von 9 mit Chlor bei 0°C in Dichlormethan synthetisiert werden. Ausbeute 96%.

3,4-Bis(diphenylphosphino)-1,6-diphenylhexan (11) :

11 wird durch Umsetzung doppeltmolarer Menge Natriumdiphenylphosphid in Dioxan mit 10, gelöst in Tetrahydrofuran, bei Raumtemperatur mit 74% Ausbeute synthetisiert [analog 1,2-Bis(diphenylphosphino)ethan ; siehe K. Issleib und D.-W. Müller, Chem. Ber. 92, 3175 (1959)].

[3,4-Bis-(diphenylphosphino)-1,6-diphenylhexan]-rhodium(I)-chlorid (12) :

Eine Lösung von 4 mmol 11 in Benzol wird unter Rühren zu einer Lösung von 1 mmol Dichlorotetracarbonyldirhodium in Benzol getropft, wobei der Komplex 12 ausfällt. Ausbeute 97%. Vgl. Synthese von [1,2-Bis(diphenylphosphino)ethan] rhodium(I)-chlorid ; A. Sacco et al., J. Chem. Soc. (London) 3274 (1964).

Katalysator Nr. 4

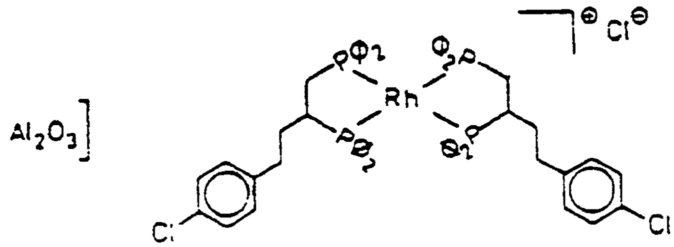

10.0 g aktivierte Aluminiumoxidkugeln (99% $Al_2O_3$) mit einem Durchmesser von 3 mm, einer inneren Oberfläche nach BET von 125 $m^2/g$ und einem Porenvolumen von 0.9 ml/g wurden mit 50 ml einer Lösung aus 117.6 mg des Rhodiumkomplexes 8 in Dichlormethan versetzt. Die gelbe Suspension wurde 15 h auf Rückflußtemperatur erhitzt, wobei sich das Lösemittel vollständig entfärbte. Nach Abziehen des Dichlormethans unter reduziertem Druck wurde der Katalysator bei 0.1 mbar und 150°C 6 h getrocknet.

| | |
|---|---|
| Charakterisierung : | Gelbe Kugeln |
| Rh-Gehalt : | 0.1 Gew% |

**Patentansprüche**

1. Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400°C und Drücken von 1-150 bar, wobei im Trägerkatalysator das Trägermaterial eine Edelmetall-Chelatverbindung trägt, die aus einer Edelmetallverbindung aus der Gruppe VIII des Periodensystems und einem Chelator mit Organostickstoff-, Organophosphor-, Organoarsen-, Organoschwefel- oder Mercaptogruppen gebildet ist, dadurch gekennzeichnet, daß der Chelator in seinem Grundkörper mit Alkyl-, Aryl- oder Aralkylgruppen substituiert ist und eine der folgenden Strukturformeln aufweist :

a) 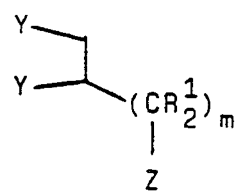

b) 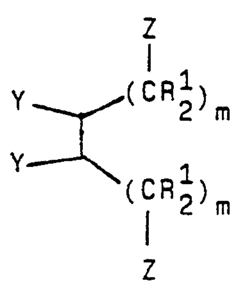

wobei

Y = $—NR^2_2$ ein stickstoffhaltiger Arylrest, $—PR^2_2$, $—AsR^2_2$ $—SR^2$ oder $—SH$ ;

Z = —H, Aryl, Phenyl (ggf. ortho-, meta- oder parasubstituiert)

$R^1$ = —H, $C_1$ bis $C_3$-Alkyl ;

$R^2$ = $C_1$ bis $C_6$-Alkyl, $C_5$ oder $C_6$-Cycloalkyl, —$C_6H_5$ oder $C_6H_5CH_2$—, die ggf. mit Halogen-, Methoxy-, Ethoxy- oder $C_1$ bis $C_3$-Alkylgruppen substituiert sind ;

m = 2-6, vorzugsweise 2-4.

2. Verfahren nach Anspruch 1, dadurch gekennzeichnet, daß im Trägerkatalysator das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator nach Anspruch 1 gebildet ist.

3. Verfahren nach Anspruch 1 oder 2, dadurch gekennzeichnet, daß der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält.

4. Verfahren nach einem der Ansprüche 1-3, dadurch gekennzeichnet, daß der Trägerkatalysator ein anorganisches oxidisches Trägermaterial oder einen Aktivkohleträger enthält.

5. Verfahren nach einem der Ansprüche 1-4, dadurch gekennzeichnet, daß der Trägerkatalysator zusammen 0,01 bis 50 Gew%, vorzugsweise 0,1 bis 20 Gew%, Chelatverbindungen und ggf. Nichtedelmetallverbindungen enthält.

6. Verfahren nach einem der Ansprüche 1-5, dadurch gekennzeichnet, daß der Trägerkatalysator in einer Korngröße von 1 bis 20 mm eingesetzt wird.

## Claims

1. A process for the preparation of monocarboxylic anhydrides of the general formula $(RCO)_2O$ by reaction of a carboxylic ester or dialkyl ether of the general formula RCOOR or ROR, in which R in each case denotes the same alkyl radical having 1 to 4 carbon atoms, with carbon monoxide in the gas phase in the presence of iodine or bromine or compounds thereof as reaction promoter and also in the presence of a supported catalyst at temperatures from 130 to 400°C and pressures of 1-150 bar, the support material in the supported catalyst supporting a noble metal chelate compound which is formed from a noble metal compound from group VIII of the periodic table and a chelating agent containing organonitrogen, organophosphorus, organoarsenic, organosulfur or mercapto groups, wherein the chelating agent is substituted in its basic structure by alkyl, aryl or aralkyl groups and has one of the following structural formulae :

a) 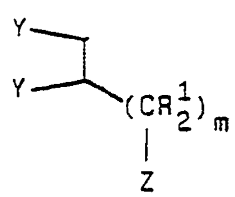

b) 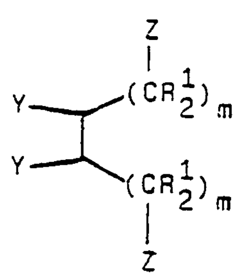

in which

Y is —$NR^2_2$, a nitrogen-containing aryl radical, —$PR^2_2$, —$AsR^2_2$, —$SR^2$ or —SH ;

Z is —H, aryl, phenyl (if appropriate, ortho-, meta- or parasubstituted)

$R^1$ is —H, $C_1$ to $C_3$-alkyl ;

$R^2$ is $C_1$ to C6-alkyl, $C_5$ or $C_6$-cycloalkyl, —$C_6H_5$ or $C_6H_5CH_2$—, which may be substituted by halogen,

methoxy, ethoxy or $C_1$ to $C_3$-alkyl groups ;

m is 2-6, preferably 2-4.

2. The process as claimed in claim 1, wherein the support material in the supported catalyst additionally supports a base metal chelate compound which is formed from a base metal compound from subgroup 6 or 8 of the periodic table of the elements and a chelating agent as claimed in claim 1.

3. The process as claimed in claim 1 or 2, wherein the supported catalyst additionally contains base metal compounds from main groups 1 to 3 or subgroups 4 to 6 or 8 of the periodic table of the elements as promoters.

4. The process as claimed in any of claims 1-3, wherein the supported catalyst contains an inorganic oxidic support material or an activated carbon support.

5. The process as claimed in any of claims 1-4, wherein the supported catalyst altogether contains 0.01 to 50% by weight, preferably 0.1 to 20% by weight, of chelate compounds and optionally base metal compounds.

6. The process as claimed in any of claims 1-5, wherein the supported catalyst is used in a particle size of 1 to 20 mm.

## Revendications

1. Procédé pour la fabrication d'anhydrides d'acides monocarboxyliques de formule générale $(RCO)_2O$ par réaction d'un ester d'acide carboxylique ou d'un oxyde de dialkyle de formules générales RCOOR ou ROR, dans lesquelles R représente chaque fois le même reste alkyle en $C_1$-$C_4$, avec le monoxyde de carbone en phase gazeuse en présence d'iode, de brome ou de leurs composés comme promoteur de réaction et en présence d'un catalyseur sur support à des températures de 130 à 400°C et sous des pressions de 1-150 bar, la matière de support dans le catalyseur sur support portant un chélate de métal noble qui est formé d'un composé de métal noble du groupe VIII de la classification périodique et d'un agent chélatant ayant des groupes organoazotés, organophosphorés, organoarséniés, organosoufrés ou mercapto, caractérisé en ce que l'agent chèlatant est substitué dans son corps de base par des groupes alkyles, aryles ou aralkyles et prèsente l'une des formules développées suivantes :

a) 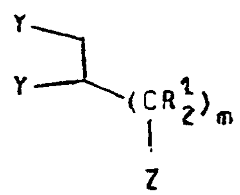

b) 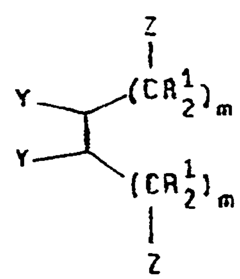

dans lesquelles

Y = $-NR^2_2$, un reste aryle azoté, $-PR^2_2$, $-AsR^2_2$, $-SR^2$ ou $-SH$ ;

Z = —H, aryle, phényle, (éventuellement ortho-, méta- ou parasubstitué)

$R^1$ = —H, alkyle en $C_1$-$C_3$ ;

$R^2$ = alkyle en $C_1$-$C_6$, cycloalkyle en $C_5$ ou $C_6$, $-C_6H_5$ ou $C_6H_5CH_2-$, qui sont éventuellement substitués par des halogènes ou des groupes méthoxy, éthoxy ou alkyles en $C_1$-$C_3$ ;

m = 2-6, de préférence 2-4.

2. Procédé selon la revendication 1, caractérisé en ce que la matière de support dans le catalyseur sur

support porte en outre un chélate de métal non noble, qui est formé d'un composé de métal non noble du 6e ou 8e sous-groupe de la classification périodique des éléments et d'un agent chélatant selon la revendication 1.

3. Procédé selon la revendication 1 ou 2, caractérisé en ce que le catalyseur sur support contient en outre des complexes de métaux non nobles des 1e au 3e groupes principaux ou des 4e à 6e ou 8e sous-groupes de la classification périodique des éléments comme promoteurs.

4. Procédé selon l'une des revendications 1 à 3, caractérisé en ce que le catalyseur sur support contient une matière de support du type oxyde inorganique ou un support de charbon actif.

5. Procédé selon l'une des revendications 1 à 4, caractérisé en ce que le catalyseur sur support contient 0,01 à 50% en poids, de préférence 0,1 à 20% en poids de chélates, et éventuellement de composés de métaux non nobles.

6. Procédé selon l'une des revendications 1 à 5, caractérisé en ce que le catalyseur sur support est utilisé à une grosseur de grain de 1 à 20 mm.